(19) Europäisches Patentamt
European Patent Office
Office européen des brevets

(11) **EP 3 533 390 A1**

(12) **EUROPEAN PATENT APPLICATION**

(43) Date of publication:
**04.09.2019 Bulletin 2019/36**

(21) Application number: **18159222.1**

(22) Date of filing: **28.02.2018**

(51) Int Cl.:
*A61B 5/021* (2006.01)   *A61B 5/1455* (2006.01)
*A61B 5/22* (2006.01)   *A61B 5/00* (2006.01)
*A61B 5/11* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **DE HAAN, Gerard
5656 AE Eindhoven (NL)**
• **MENA BENITO, Maria Estrella
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **REHABILITATION SYSTEM AND METHOD**

(57)    A rehabilitation system uses a sensor to monitor a parameter which varies with blood pressure and to estimate an effort of a subject while performing a rehabilitation task using this parameter monitoring. It can then be determined when a difficulty level of the rehabilitation task should be changed because subject is able to perform easily the existing rehabilitation task. This enables automated generation of an adaptive rehabilitation program.

FIG. 1

# EP 3 533 390 A1

**Description**

FIELD OF THE INVENTION

This invention relates to a rehabilitation system and method, in particular for ensuring that appropriate rehabilitation exercises are selected for a subject.

BACKGROUND OF THE INVENTION

Rehabilitation, or physical therapy, promotes mobility and function after an injury or medical condition. For example, rehabilitation is frequently used after musculoskeletal injuries and for the management of long-term musculoskeletal conditions. Rehabilitation can improve the quality of life of a subject.

Although dependent on the particular diagnosis of a subject, rehabilitation typically involves specific training exercises, e.g. power, mobility, resistance or balance training. Resistance training is a common part of musculoskeletal rehabilitation of subjects that suffer from various injuries.

Typically, the training difficulty starts at a low level that is easily achievable and increases regularly until an acceptable performance level is achieved. The increases should not be taken too early, as this can exacerbate an injury. The increases should not be delayed unnecessarily either, as this can delay recovery.

A problem with rehabilitation programs is that the decision making process is rather subjective and often involves visiting a rehabilitation expert for guidance. A consequence of this is increased cost and inconvenience for the subject.

It would be desirable to provide a way to automate the monitoring of the progress of a subject and also quantify their performance during rehabilitation. This would enable a subject to carry out an effective rehabilitation program without the need for expert supervision.

An optimal training program will not only increase the likely compliance of a subject to the training program, but also enable recovery in a faster and more effective way, thus reducing medical costs, and/or improving outcomes.

SUMMARY OF THE INVENTION

The invention is defined by the claims.

According to examples in accordance with an aspect of the invention, there is provided a rehabilitation system, comprising:

a sensor for outputting at least one representation of blood pressure from a subject;
a processing unit adapted to:

estimate the effort of a subject while performing a rehabilitation task based on the representation of blood pressure; and
determine when a difficulty of the rehabilitation task should be changed based on a change in effort level required for the rehabilitation task.

This rehabilitation system estimates the effort required for a subject to perform a task. The task may be a part of a resistance training program for a subject in physical rehabilitation. This rehabilitation for example relates to a damaged body part, such as an arm, leg, hand, foot, or any joint (shoulder, elbow, wrist, hip, knee, ankle). The estimation of effort uses a representation of blood pressure (i.e. a measure of blood pressure or a signal which depends on blood pressure but not necessarily in a directly proportional way) to indicate when the level of resistance can be increased (i.e. the task can be made more difficult). The representation of blood pressure can be obtained by any suitable known sensor.

The invention is based on the effect that the mental effort of the subject required to perform a physical task raises the blood pressure of the subject. This mental effort is thus related to the physical difficulty in performing the task. The measured effect is thus a mental effect rather than a direct physical effect. By determining when the difficulty of the task should be changed in an automated way, the need for constant medical supervision during the rehabilitation process is removed.

The sensor for example comprises a PPG sensor. This may be a contact PPG sensor or a non-contact remote PPG sensor such as a camera-based sensor.

The processing unit is for example adapted to compare the estimated effort level with a reference effort level which represents an expected post-rehabilitation effort level either for the same task or a related task. In this way, when the difficulty in performing a given task approaches or reaches the expected post-rehabilitation effort level, the task difficulty may be increased. The expected post-rehabilitation level for the task being performed may be the actual reference effort level, or it may be extrapolated from the reference level. Thus, the reference effort level may relate to a different task difficulty and/or a different muscle group.

The processor may be adapted to determine an expected post-rehabilitation effort level from a calibration procedure. In this way, the estimation of effort level is tailored to the subject.

The rehabilitation is for example for a first, damaged, body part of the subject, and the processor is adapted to implement the calibration procedure by estimating an effort level for the subject when performing the rehabilitation task using a second, non-damaged, body part of the subject. By performing the task with a non-damaged body part, the reference is set taking into account the subject's own physiological parameters.

The body parts do not need to be the same. For example, the effort level as experienced when performing the task with one body part (e.g. leg) may be converted to a corresponding level when performing the task with another body part (e.g. arm). Thus, the comparison with the reference effort may involve a conversion process to convert from one body part to another or from one task level to another.

Preferably, the first body part and the second body part the left and right body parts of the same type, for example the two hands, feet, arms, legs, shoulders, elbows, knees or hips.

In an alternative approach, the processor may be adapted to determine the expected post-rehabilitation level from a database of anatomical information.

The system preferably comprises a display, and the processor is adapted to control the display to output information relating to a suitable difficulty of the rehabilitation task.

According to another aspect of the invention, there is provided a rehabilitation method, comprising:

obtaining at least one representation of blood pressure from a subject;
estimating the effort of a subject while performing a rehabilitation task by monitoring the representation of blood pressure during the rehabilitation task; and
determining when a difficulty of the rehabilitation task should be changed based on a change in effort level for the rehabilitation task.

The method may comprise using a PPG sensor to provide the representation of blood pressure. The estimated effort level may be compared with a reference effort level which represents an expected post-rehabilitation level.

The rehabilitation is for example for a first, damaged, body part of the subject, and wherein the method comprises determining an expected post-rehabilitation level from:

a calibration procedure which involves estimating an effort level for the subject when performing the rehabilitation task using a second, non-damaged, body part of the subject; or
from a database of anatomical information.

The invention may be implemented at least in part in software, and thus provides a computer program comprising computer program code means, which is adapted, when said program is run on a computer, to implement the method defined above.

BRIEF DESCRIPTION OF THE DRAWINGS

Examples of the invention will now be described in detail with reference to the accompanying drawings, in which:

Figure 1 shows a schematic embodiment of the system;
Figures 2 and 3 are graphical results of the systolic and diastolic blood pressure of a subject while performing different exercise tasks, respectively; and
Figure 4 shows a method for assisting a subject in a rehabilitation process.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a rehabilitation system which uses a sensor to monitor a parameter which varies with blood pressure and to estimate an effort of a subject while performing a rehabilitation task using this parameter monitoring. It can then be determined when a difficulty level of the rehabilitation task should be changed because the subject is able to perform easily the existing rehabilitation task. This enables automated generation of an adaptive rehabilitation program.

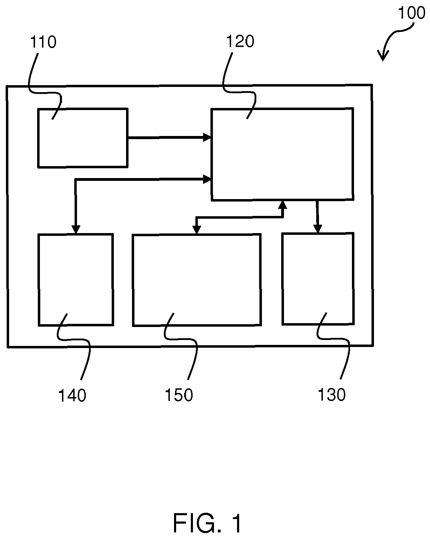

Figure 1 shows a rehabilitation system 100 in block diagram form.

The rehabilitation system 100 comprises a sensor 110 and a processing unit 120. A display 130 provides an output to the user of the system. The system also includes a memory 140 and a user interface 150. The sensor 110 receives a representation of a subject's blood pressure, or a signal which varies with blood pressure. In a preferred embodiment, a photoplethysmography (PPG) sensor is used.

A PPG sensor is a pulse oximeter. The purpose of pulse oximetry is to monitor the oxygen saturation of a subject's blood. While the purpose of such a sensor is to obtain a measure of blood oxygen saturation, it also detects changes in blood volume in the skin, and thereby performs PPG sensing. By detecting changes in blood volume, a cyclic signal corresponding to the pulse is obtained. PPG sensors, such as pulse oximeters, are thus commonly used to provide a measure of the pulse rate.

It is well known to combine a pressure cuff with PPG measurement to derive a measure of blood pressure. WO 2018/019742 discloses a PPG sensor with an integrated force transducer which either applies a force or measures a force which may be manually applied by the user. The PPG sensor is a contact-based PPG arrangement.

A less invasive approach would avoid the need for a pressure cuff or other force transducer. It has been found that the shape of the PPG signal provides an indirect measure of blood pressure. A change in blood pressure causes a change in the AC component and DC component of a PPG signal.

A first example of this effect is disclosed by Monte-Moreno E, Non-invasive estimate of blood glucose and blood pressure from a photoplethysmograph by means of machine learning techniques, Artif. Intell. Med. 2011 Oct;53(2):127-38. It discloses a simultaneous non-invasive estimate of the blood glucose level (BGL) and the systolic (SBP) and diastolic (DBP) blood pressures, using a PPG sensor and machine learning techniques. The system operates based on the principle that there is functional relationship between the shape of the PPG waveform and the blood pressure and glucose levels.

Another example is disclosed by Xiaoman Xing and Mingshan Sun, Optical blood pressure estimation with photoplethysmography and FFT-based neural networks, Biomed. Opt. Express. 2016 Aug 1; 7(8): 3007-3020. Beat-to-beat optical blood pressure estimation is achieved using only PPG signals. Amplitudes and phases of cardiac components are extracted by a fast Fourier transform and are used to train an artificial neural network, which is then able to estimate blood pressure from the PPG.

Arterial stiffness measurements may also be made to provide a representation of blood pressure from a subject. An example of an arterial stiffness estimation is disclosed by A.V. Moço, L.Z. et. al., Camera-based assessment of arterial stiffness and wave reflection parameters from neck micro-motion, Physiol. Meas. 38, no. 8, July, 2017, pp. 1576-1598.

Alternatively, two PPG sensors may be used to measure changes in pulse wave velocity (PWV). Multi-site PPG analysis is for example disclosed in WO 2015/078735. The PPG signals are obtained by non-contact camera-based monitoring in that proposed system.

It is known that a continuous, non-invasive and indirect measurement of blood pressure is possible based on changes in pulse wave velocity, which can be determined from multiple PPG measurements. PWV is the speed of a pressure pulse propagating along the arterial wall. Typically, a relation of blood pressure and PWV in arteries is expressed by the Moens-Korteweg-relation, which can be derived from hydrodynamic theory:

$$c = \sqrt{\frac{hE_t}{2\rho R}} \qquad\qquad (\text{Eq. 1})$$

This is often used to describe the relation of pulse-wave-velocity and blood pressure. Here: c=pulse wave velocity, $E_t$=tangential elasticity module, $\rho$=density, R=radius of artery, h=artery wall thickness.

PWV can be calculated from the pulse transit time (PTT) which is the time a propagating wave takes on the same cardiac cycle between two separate arterial sites. PTT has been shown to be quasi-linear to low BP values, but increases exponentially at higher pressures.

For example a known set-up for measuring PTT is to use an ECG sensor and a PPG sensor. The PTT is given by the time difference between the R-peak and characteristic points in PPG. The PPG can be measured at various positions on the body e.g. ear or finger.

A PPG sensor contains at least one LED, and one light sensor. The LED and sensor are placed such that the LED directs light into the skin of the user, which is reflected or transmitted, and detected by the sensor. The amount of reflected/transmitted light is determined by, amongst others, the perfusion of blood within the skin.

The PPG system for example includes a red LED, a near-infrared LED, and a photodetector diode. The sensor is typically configured with the LEDs and photodetector diode directly on the skin of the subject, typically on a digit (finger or toe) or earlobe.

Other places on the subject may also be suitable, including the forehead, the nose or other parts of the face, the wrist, the chest, the nasal septum, the alar wings, the ear canal, and/or the inside of the mouth, such as the cheek or the tongue.

The LEDs emit light at different wavelengths, which light is diffused through the vascular bed of the skin and received by the photodetector diode. The changing absorbance at each of the wavelengths is measured, allowing the sensor to determine the absorbance due to the pulsing arterial blood alone, excluding venous blood, skin, bone, muscle, and fat for example. The resulting PPG signal may then be analyzed.

Other simpler versions of a system for obtaining PPG data may be used, including a version with a single light source of one or more wavelengths. The absorption or reflectance of the light is modulated by the pulsatile arterial blood volume and detected using a photodetector device.

In transmissive pulse oximetry, a sensor device is placed on a thin part of the body of the subject. Reflectance pulse oximetry may be used as an alternative to transmissive pulse oximetry. This method does not require a thin section of the person's body and is therefore well suited to more universal application such as the feet, forehead and chest.

A basic design of a PPG sensor 110 for example is a contact sensor with a single wavelength light source, e.g. green light (550nm) to measure the PPG signal from a relatively stable body-part, like the forehead. The light source is pulsed with a certain light output frequency such as 128Hz. A sampling frequency of the optical sensor is higher, for example 256Hz so that it measures during light source activation and between light source activations. This allows the system to distinguish between the emitted light from the LED and the ambient light, and thereby filter out the ambient light from the signal received during a light source pulse.

In other known proposals, PPG data can be obtained from camera images, where ambient light and / or additional light sources are used to illuminate the tissue, such as skin. PPG measurements can thus even be carried out at a distance from the tissue, where the light source and/or detector are not in contact with the tissue, such as in the case of camera-based measurements.

The PPG data may be obtained at one or more wavelengths, such as any number of wavelengths typically between 1 and 10, but more than 10 wavelengths may even be used.

Apparatus and techniques for obtaining PPG data are well known in the art and indeed many different PPG sensors are commercially available. They are for example used in devices for measuring heart rate during exercise.

This invention is based on associating changes in blood pressure with differences in mental effort required to perform a rehabilitation task. In particular, the processing unit performs an estimation of the effort of the subject while performing a rehabilitation task. This effort level is monitored over time and it is then determined when a difficulty of the rehabilitation task should be changed based on a change in effort level for the rehabilitation task. This rehabilitation for example comprises resistance exercises to be performed by a damaged body part, such as an arm, leg, hand, foot, or any joint (shoulder, elbow, wrist, hip, knee, ankle). The estimation of effort indicates when the level of resistance can be increased (i.e. the task can be made more difficult). The level of resistance is for example an amount of weight being used when performing a particular exercise.

The processing unit 120 thus monitors the subject over time while carrying out a rehabilitation task. The monitoring of the subject includes continually processing the output from the sensor 110.

The change in effort is for example visible in the PPG signal as an increase in AC variation in the PPG signal. Similarly, a change in pulse transit time may be monitored. Although there is no direct simple relationship between blood pressure and strength of the PPG signal or pulse transit time, the relative change from a baseline (before an attempted exercise) is an excellent metric which correlates with the mental effort required to perform a physical task.

A reasonable effort using a healthy body part causes an increase in PPG signal amplitude, either the AC or the DC component, due to the increase in blood pressure. A measure of effort is thus derived from the analysis of the PPG signals.

If the subject has an injured right arm, the subject can lift a weight with the left arm as part of a calibration routine. The PPG sensor 110 then outputs a signal which is dependent on the blood pressure and the processing unit 120 calculates an estimation of effort based on the increase in PPG amplitude signal from the previous baseline level i.e. before the physical exertion. This provides a reference effort level.

The subject then repeats the exercise with the injured arm. The same weight or a lighter weight may be used. If a different weight is used, the reference effort level is converted before it is used as a comparison reference. The system then stores the current progress and other relevant data, such as the sensor output, in the memory 140. The processing unit 120 continues to monitor the subject during the repeated exercise.

In one example, the weight used for the calibration is a relatively heavy weight for the subject, for example representing the weight which that use would like to achieve post-rehabilitation with the injured arm. The starting weight chosen for the rehabilitation exercise is then a lighter weight, but which causes a corresponding amount of effort.

Thus, although the injured arm is lifting a lighter weight, the same estimated effort as a healthy arm, lifting a heavier weight, is achieved. At the point which the estimated effort when using the injured arm falls, the system indicates to the user on the display 130 that it is advisable to increase the difficulty (weight) of the exercise. The process continues until the estimated effort for a given weight is the same for both arms.

The progress and current difficulty of the task, based on the calculated effort from the processing unit 120, can also be output to the display 130 by the processing unit 120 at any time.

In some embodiments, the estimated effort, or recent effort history, may be shared (or transferred) to a medical professional to adapt the rehabilitation exercises as progress is made through a program. The data can be accessed by means of the interface 150. The interface 150 can be in the form of a serial bus or some communication device, wireless or wired, that is appropriate for communicating with another device or system and transferring data. Such data can be transferred by methods already known such as: Wi-Fi (RTM), Bluetooth (RTM), cellular communication (2G/3G/4G LTE), or USB (RTM).

The automated indication of suitable exercises gives a subject the information necessary to make progress in a rehabilitation, or strength training program, without the need for an appointment with a medical expert and the costs associated with that appointment.

In many uses of the system, the left-right symmetry of the body will be advantageously utilized to perform a subject-specific calibration, as explained above. However, in embodiments where the left-right symmetry cannot be relied upon, statistics about the relative strength of different muscles can be used. The statistics can be either known in the art or acquired in advance. An initial exercise may, for example, involve the use of a hand grip, while a second (rehabilitation) exercise is to lift a weight using a leg. In this case, the required statistics link the typical strength of a hand with the typical strength of the leg to specify the progress of the rehabilitation exercise.

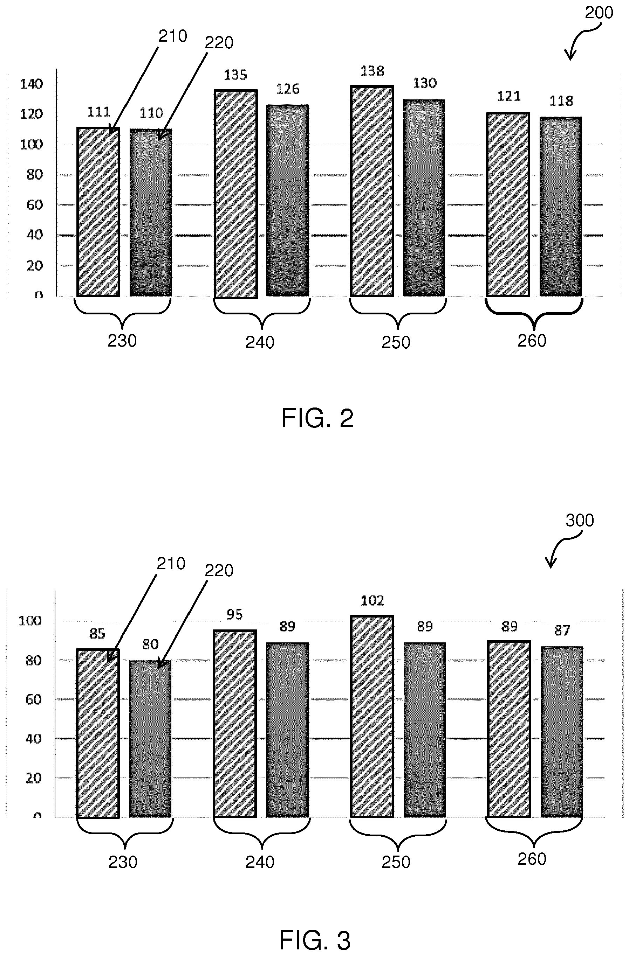

Figures 2 and 3 show the systole and diastole blood pressures measured as part of an experiment to demonstrate the functioning of the system.

A pilot test was performed on a volunteer with an injured left arm, i.e. weaker due to past fracture, and a healthy right arm. The pilot test was performed in order to verify the assumptions relating effort, injury, and blood pressure.

Figure 2 shows a plot 200 of the systolic blood pressure of the volunteer as a function of arm effort. In particular, the arm effort chosen was the lifting of a weight of various masses. In each pair of bar measurements the left bar represents the injured arm measurement 210 and the right bar indicates the healthy arm measurement 220. The injured arm and healthy arm were made to hold weights of 0kg (plots 230), namely free lifting of the arm, 2kg (plots 240), 3kg (plots 250) and a final 0kg (plots 260).

As shown in Figure 2, the pilot test indicates that the systolic blood pressure rises as a function of the effort of the subject rather than solely based on physical work done. Consequently, the blood pressure rises more when lifting the same weight with the injured arm than when lifting it with the health arm.

Figure 3 shows a graph 300 of the diastolic blood pressure as a function of arm effort. The individual parts of the graph are the same as described above in Figure 2. Thus Figure 3 shows pairs of plots for 0kg, 2kg, 3kg and 0kg.

As can be seen from both Figures 2 and 3, the hypothesis that blood pressure rises with effort of the subject is valid for both systolic and diastolic pressure measurements.

During the experiment the measurements were performed using an upper-arm cuff blood pressure measuring device (i.e., a sphygmomanometer), while the volunteer was seated in an office chair. In every case, the measurements of blood pressure were taken 3 times per arm on the opposite arm to the one used for the exercise.

As discussed briefly above, the results of this pilot are as expected; blood pressure increases the more effort is required, i.e. it rises with increasing weight, but more so in the injured arm. This confirmed the hypothesis that the blood pressure is a proxy for effort, rather than only physical effect (actual weight lifted).

It is noted that a precise calibration between PPG signal characteristics and blood pressure, and a precise calibration between blood pressure and effort, are not needed. However, for a particular subject, it is the comparison of the efforts required to activate different muscles including the injured muscle that is important.

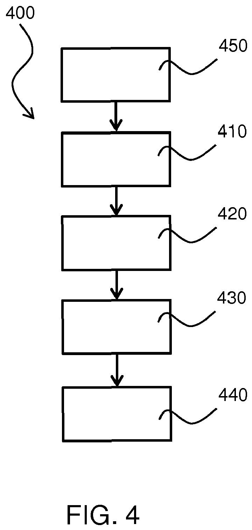

Figure 4 depicts a method for assisting a subject in a rehabilitation process.

The method 400 comprises:

in step 410, providing at least one representation of blood pressure from a subject;
in step 420, estimating the effort of a subject while performing a rehabilitation task by monitoring the representation of blood pressure during the rehabilitation task;
in step 430, comparing the estimated effort level with a reference effort level which represents an expected post-rehabilitation effort level; and
in step 440, determining when a difficulty of the rehabilitation task should be changed based on a change in effort level for the rehabilitation task.

The method may comprise a calibration step 450 during which an expected post-rehabilitation effort level is obtained either from a calibration procedure which involves estimating an effort level for the subject when performing the rehabilitation task using a second, non-damaged, body part of the subject, or else estimating using a database of anatomical information.

As mentioned above, the system may exploit the left-right symmetry of a subject's body, and compare the left and right sided efforts for a given resistance-level. The difference between the efforts required for a health body-side and an injured body-side may then be analyzed. Using such information, the rehabilitation progress may be monitored over a long period, for example, in the case of a stroke victim. In the absence of a symmetry that can be exploited, statistical data linking the relative strength of different muscles can be used for the same purpose.

The example above is based on analyzing characteristics of a single PPG signal to provide a measure which is correlated with, and therefore representative of, blood pressure. There are other measures which may be taken. For example, any measure of pulse transit time may be used to provide a representation of blood pressure. Examples include:

an ECG signal and a bio-impedance measurement at the arm (impedance plethysmography (IPG)). PTT is then given by the time-difference between an R-peak of an ECG signal and characteristic points in the IPG;
an Impedance Cardiography (ICG) signal from the thorax and a bio-impedance measurement at arm (IPG). The PTT is given by the time-difference between characteristic points in the ICG and characteristic points in the IPG; and
an Impedance plethysmogram (IPG) measurement at a first position on an arm and a bio-impedance measurement at a second position on an arm. The PTT is given by the time-difference between characteristic points in the IPG signal and characteristic points in the bio-impedance measurement.

The difficulty of a rehabilitation exercise may for example relate to a weight that is used during the exercise. It may instead or additionally relate to a number of repetitions and/or a range of movement to be followed, and/or a speed with which the task is to be carried out.

Various algorithms may be used for interpreting PPG signals to assist in making an estimation of effort. Such algorithms include:

CHROM, which linearly combines the chrominance-signals by assuming a standardized skin-color to white-balance the images;
PBV, which uses the signature of blood volume changes in different wavelengths to explicitly distinguish the pulse-induced color changes from motion noise in RGB measurements;
PCA and ICA based blind source separation, which use different criteria to separate temporal RGB traces into uncorrelated or independent signal sources to retrieve the pulse; and
2SR, which measures the temporal rotation of the spatial subspace of skin-pixels for pulse extraction.

In some examples a remote camera may be used for PPG measurement, as mentioned above. An RGB camera makes use of three wavelength-channels (RGB) to enable a motion robust remote measure of the AC-component (pulse), e.g. from the forehead of a subject.

As discussed above, the system makes use of a processing unit, which functions as the overall controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A rehabilitation system, comprising:

   a sensor (110) for outputting at least one representation of blood pressure from a subject;
   a processing unit (120) adapted to:

   estimate the effort of a subject while performing a rehabilitation task based on the representation of blood pressure; and
   determine when a difficulty of the rehabilitation task should be changed based on a change in effort level required for the rehabilitation task.

2. A system as claimed in claim 1, wherein the sensor (110) comprises a PPG sensor.

3. A system as claimed in claim 2, wherein the PPG sensor (110) comprises a camera-based sensor.

4. A system as claimed in any preceding claim, wherein the processing unit (120) is adapted to compare the estimated effort level with a reference effort level which represents an expected post-rehabilitation effort level.

5. A system as claimed in claim 4, wherein the processor (120) is adapted to determine an expected post-rehabilitation effort level from a calibration procedure.

6. A system as claimed in claim 5, wherein rehabilitation is for a first, damaged, body part of the subject, and wherein the processor is adapted to implement the calibration procedure by estimating an effort level for the subject when performing a rehabilitation task using a second, non-damaged, body part of the subject.

7. A system as claimed in claim 6, wherein the first body part and the second body part the left and right body parts of the same type.

8. A system as claimed in claim 4, wherein the processor (120) is adapted to determine the expected post-rehabilitation effort level from a database of anatomical information.

9. A system as claimed in any preceding claim, further comprising a display (130), wherein the processor is adapted to control the display to output information relating to a suitable difficulty of the rehabilitation task.

10. A rehabilitation method, comprising:

    (410) obtaining at least one representation of blood pressure from a subject;
    (420) estimating the effort of a subject while performing a rehabilitation task by monitoring the representation of blood pressure during the rehabilitation task; and
    (440) determining when a difficulty of the rehabilitation task should be changed based on a change in effort level for the rehabilitation task.

11. A method as claimed in claim 10, comprising using a PPG sensor (110) to provide the representation of blood pressure.

12. A method as claimed in claim 10 or 11, comprising (430) comparing the estimated effort level with a reference effort level which represents an expected post-rehabilitation effort level.

13. A method as claimed in claim 12, wherein rehabilitation is for a first, damaged, body part of the subject, and wherein the method comprises determining an expected post-rehabilitation effort level from:

    a calibration procedure (450) which involves estimating an effort level for the subject when performing the rehabilitation task using a second, non-damaged, body part of the subject; or
    a database of anatomical information.

14. A method as claimed in any one of claims 10 to 13, further comprising (440) outputting information relating to a suitable difficulty of the rehabilitation task.

**15.** A computer program comprising computer program code means, which is adapted, when said program is run on a computer, to implement the method of any one of claims 10 to 14.

FIG. 1

FIG. 2

FIG. 3

FIG. 4

Europäisches
Patentamt

European
Patent Office

Office européen
des brevets

# EUROPEAN SEARCH REPORT

Application Number

EP 18 15 9222

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/135627 A1 (YUAN ZUO [CN]) 18 May 2017 (2017-05-18) | 1-12,14, 15 | INV. A61B5/021 |
| Y | * paragraphs [0042] - [0056] * | 13 | A61B5/1455 A61B5/22 |
| X | US 2015/287187 A1 (REDTEL HOLGER [DE]) 8 October 2015 (2015-10-08) | 1-12,14, 15 | A61B5/00 A61B5/11 |
| Y | * paragraphs [0003], [0004], [0044] - [0050], [0058] - [0060], [0085] - [0099], [0102] - [0104] * | 13 | |
| X | US 2015/348429 A1 (DALAL EDUL N [US] ET AL) 3 December 2015 (2015-12-03) | 1-12,14, 15 | |
| Y | * paragraphs [0045] - [0052], [0066] - [0068] * | 13 | |
| Y | US 2011/196262 A1 (MCLEOD KENNETH J [US] ET AL) 11 August 2011 (2011-08-11) * paragraphs [0248], [0249] * | 13 | |
| Y | US 2017/251955 A1 (GOSSLING MARTIN [GB]) 7 September 2017 (2017-09-07) * paragraph [0044] * | 13 | TECHNICAL FIELDS SEARCHED (IPC)<br><br>A61B |

The present search report has been drawn up for all claims

| Place of search | Date of completion of the search | Examiner |
|---|---|---|
| Berlin | 10 August 2018 | Trachterna, Morten |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 15 9222

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2018

| Patent document cited in search report | | Publication date | Patent family member(s) | | Publication date |
|---|---|---|---|---|---|
| US 2017135627 | A1 | 18-05-2017 | CN | 104814728 A | 05-08-2015 |
| | | | EP | 3305184 A1 | 11-04-2018 |
| | | | US | 2017135627 A1 | 18-05-2017 |
| | | | WO | 2016188001 A1 | 01-12-2016 |
| US 2015287187 | A1 | 08-10-2015 | BR | 112015010677 A2 | 11-07-2017 |
| | | | CA | 2891194 A1 | 15-05-2014 |
| | | | CN | 105338890 A | 17-02-2016 |
| | | | EP | 2916724 A1 | 16-09-2015 |
| | | | JP | 2016501048 A | 18-01-2016 |
| | | | KR | 20150095661 A | 21-08-2015 |
| | | | RU | 2015122420 A | 10-01-2017 |
| | | | US | 2015287187 A1 | 08-10-2015 |
| | | | US | 2018122073 A1 | 03-05-2018 |
| | | | WO | 2014072461 A1 | 15-05-2014 |
| US 2015348429 | A1 | 03-12-2015 | NONE | | |
| US 2011196262 | A1 | 11-08-2011 | NONE | | |
| US 2017251955 | A1 | 07-09-2017 | EP | 3200693 A1 | 09-08-2017 |
| | | | GB | 2530754 A | 06-04-2016 |
| | | | GB | 2551238 A | 13-12-2017 |
| | | | JP | 2017529929 A | 12-10-2017 |
| | | | US | 2017251955 A1 | 07-09-2017 |
| | | | WO | 2016051162 A1 | 07-04-2016 |

EPO FORM P0459

For more details about this annex : see Official Journal of the European Patent Office, No. 12/82

14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018019742 A

- WO 2015078735 A

**Non-patent literature cited in the description**

- *Artif. Intell. Med.,* October 2011, vol. 53 (2), 127-38
- *Biomed. Opt. Express.,* 01 August 2016, vol. 7 (8), 3007-3020

- **A.V. MOÇO, L.Z.** Camera-based assessment of arterial stiffness and wave reflection parameters from neck micro-motion. *Physiol. Meas.,* 08 July 2017, vol. 38, 1576-1598